# EUROPEAN PATENT SPECIFICATION

(11) **EP 3 773 450 B1**
(45) Date of publication and mention of the grant of the patent: **15.03.2023**
(21) Application number: 19721141.0
(22) Date of filing: 09.04.2019
(51) Int. Cl.: A61K 8/38, A61K 8/46, A61K 8/49, A61Q 11/00, A61K 8/20, A61K 8/22

(54) **TEETH-WHITENING KITS AND RELATED METHODS OF WHITENING TEETH**
KIT UND VERFAHREN ZUR ZAHNBLEICHUNGUNG
KIT ET MÉTHODE DE BLANCHIMENT DES DENTS

(30) Priority: 09.04.2018 IT 201800004320
(43) Date of publication of application: 17.02.2021
(73) Proprietor: IDS RESEARCH SRL, 16122 Genova (IT)
(72) Inventor: SANTONE, Claudio, 63074 San Benedetto Del Tronto (Ascoli Piceno) (IT)
(74) Representative: Cutropia, Gianluigi
(86) International application number: PCT/IB2019/052894
(87) International publication number: WO 2019/197977

(56) References cited:
- WO-A1-97/21418
- WO-A1-2011/084744
- WO-A1-2011/134087
- WO-A2-2014/042936
- PREDRAG BANKOVI ET AL: "Al,Fe-pillared clays in catalytic decolorization of aqueous tartrazine solutions", APPLIED CLAY SCIENCE, ELSEVIER, AMSTERDAM, NL, vol. 58, 23 January 2012 (2012-01-23), pages 73-78, XP028473817, ISSN: 0169-1317, DOI: 10.1016/J.CLAY.2012.01.015 [retrieved on 2012-01-30]

## Description

### TECHNICAL FIELD

The present invention is in the field of teeth-whitening kits comprising oxidizing compositions and teeth-whitening methods employing such kits.

### BACKGROUND OF THE DISCLOSURE

Various agents such as certain foods and tobacco, the process of aging, diseases, trauma, medications, some congenital conditions, and environmental effects can cause teeth to become discolored. Because white or whitened teeth are usually considered to be cosmetically desirable, there is a great deal of interest in developing compositions and methods for whitening teeth.

Peroxide and peroxyacid compounds, such as hydrogen peroxide and carbamide peroxide, are known to be useful as bleaching agents in teeth whitening compositions. Appropriate heat, light, or chemical sources can accelerate the release of oxygen radicals from the peroxide or peroxyacid compounds.

Tooth sensitivity following treatment, and the time required for teeth whitening compositions (typically requiring about an hour of time or multiple applications or both), however, remain a significant drawback.

WO 2014/042936 A2 discloses a method for whitening teeth comprising: a) providing a composition comprising an oxidizing agent (hydrogen peroxide, carbamide peroxide) and at least one chromophore (Eosin Y, Fluorescein, Rose Bengal, Erythrosine); b) applying the composition to at least one tooth; and c) exposing the composition on the at least one tooth to actinic light (emitting light of wavelength 250-700 nm, preferably 400-500 nm).

The present invention is directed to improvements in teeth-whitening kits and further provides methods of using the same.

### SUMMARY OF THE INVENTION

In a first aspect, the present invention provides a kit for teeth whitening. The teeth-whitening kit of the invention comprises a first and a second composition, wherein the first composition comprises an oxidizing agent and the second composition comprises at least two dyes as specified below and an oxidation catalyst.

In certain embodiments, the oxidizing agent of the first composition comprises hydrogen peroxide or carbamide peroxide or both. In certain embodiments, the first component is an aqueous solution of hydrogen peroxide, wherein the hydrogen peroxide concentration is of about 50%. In other embodiments, the hydrogen peroxide concentration is less than or equal to 6% by weight.

In certain embodiments, the oxidizing agent in the first composition is carbamide peroxide, preferably in the form of a gel having a carbamide peroxide concentration of about 29%. In other embodiments, the total content of the oxidizing agent in the first composition is equivalent to about 6% by weight of hydrogen peroxide content in the composition.

The second composition comprises at least two dyes and an oxidation catalyst. The at least two dyes are Azorubine, also named as Carmoisine (E122) and Tartrazine (E102).

The second composition further comprises a catalyst to promote the decomposition of the oxydizing agent and the development of oxygen. In a preferred embodiment, the catalyst comprises potassium iodide (KI).

In certain embodiments, the second composition further comprises a stabilizing agent.

In certain embodiments, the second composition further comprises a thickening agent. In certain embodiments, the thickening agent comprises silicon dioxide and/or fumed silica having a particle size less than 1 micron.

In certain embodiments, the second composition further comprises a hydrophilic gelling agent. In certain embodiments, the hydrophilic gelling agent comprises polypropylene glycol, polyethylene glycol, propylene glycol, glycerol, or a vinyl polymer with active carboxyl groups such as carboxypolymethylene, or any combination thereof.

In certain embodiments, the kit additionally comprises a light source, such as a lamp, LED or laser.

The present invention also provides a method for whitening teeth. The method comprises mixing the first and the second compositions of a kit according to the invention to form a teeth-whitening composition, applying the teeth-whitening composition thereby obtained to at least one tooth and exposing the teeth-whitening composition on the at least one tooth to actinic light.

### DETAILED DESCRIPTION OF THE INVENTION

Features and advantages of the present invention will become more apparent in light of the following detailed description of selected embodiments.

The present invention provides kits for teeth whitening. The kits of the invention comprise a first composition comprising an oxidizing agent, and a second composition comprising at least two dyes as specified below and an oxidation catalyst. The first composition is also named "oxidizing composition". In certain embodiments, the second composition is named "dye gel composition".

An oxidizing agent is a chemical compound that readily transfers oxygen atoms and oxidizes other compounds. This term as used herein also includes precursors of compounds capable of oxidizing other compounds. Oxidizing agents can be provided in powder, liquid or gel form.

In some preferred embodiments, the oxidizing agent is selected from hydrogen peroxide, carbamide peroxide and or combinations thereof. In certain embodiments, the hydrogen peroxide content in the first composition of the kit of the invention is less than or equal to 50% by weight. In other embodiments, the hydrogen peroxide content in the first composition is less than or equal to 6% by weight. In other embodiments, the carbamide peroxide content in the first composition is less than or equal to 29% by weight. In other embodiments, the total content of the carbamide peroxide is equivalent to about 6% by weight of hydrogen peroxide content in the composition.

Advantageously, a lowered oxidizing agent content may be associated with decreased gum and tooth sensitivity, decreased dehydration of the tooth, decreased brittleness of the enamel, decreased decalcification of the enamel and longer lasting tooth whitening effects. The content of oxidizing agents such as hydrogen peroxide in teeth whitening compositions are also relevant from a regulatory perspective. For example, levels above 6% are not permitted by the European Commission (European Cosmetics Directive 767768/EEC and European Cosmetic Regulation EC 1223/2009). Therefore, the use of 6% or less hydrogen peroxide, or a hydrogen peroxide equivalent, can be achieved by means of certain embodiments of the kit of the invention without compromising teeth whitening effects or necessitating longer treatment times.

As mentioned, the second composition of the kit of the invention comprises at least two dyes and an oxidation catalyst. The dyes are Azorubine, which is also named as Carmoisine (E122) and Tartrazine (E102). Azorubine and Tartrazine are the dyes to be used in the second composition of the kit of the invention because they stable against degradation from the oxydizing agent. In some embodiments, the second composition comprises about 0.04 to 0.08 % by weight of Azorubine and about 0.04 to 0.08% by weight Tartrazine. More preferably, the second composition comprises from 0.05 to 0.06 % by weight of Azorubine and from 0.05 to 0.06 % by weight of Tartrazine.

The second composition also includes a catalyst. In a preferred embodiment, the catalyst is potassium iodide (KI), more preferably in an amount of from 90 to 180 ppm.

The second composition may also include a gelling agent, such as a hydrophilic gelling agent. "Gelling agent" means an agent that thickens and stabilizes liquid solutions, emulsions and suspensions.

In one embodiment, the gelling agent includes, for example, polypropylene glycol, polyethylene glycol, propylene glycol, glycerol, or a vinyl polymer with active carboxyl groups such as carboxypolymethylene, or any combination thereof. In a particularly preferred embodiment, the hydrophilic gelling agent comprises glycerol, propylene glycol, carboxypolymethylene (Carbopol) and water.

In one embodiment, the hydrophilic gelling agent comprises a Carbopol. Such polymers are commercially available e.g. from B.F. Goodrich under the designation Carbopol^{®} 420, 430, 475, 488, 493, 910, 934, 934P, 9 / IPNF, 974P NF, 980 NF, 981 NF and the like. Carbopols are versatile controlled-release polymers, and belong to a family of carbomers which are synthetic, high molecular weight, non-linear polymers of acrylic acid, crosslinked with polyalkenyl polyether. In some embodiments, the Carbopol is Carbopol^{®} 974P NF, 980 NF, 5984 EP, ETD 2020NF, Ultrez 10 NF, 934 NF, 934P NF, 940 NF or 71G NF.

The second composition may also include a stabilizing agent. In some embodiments, the stabilizing agent is a metal chelator, for example ethylenediaminetetraacetic acid (EDTA).

In one embodiment, the pH of the second composition is acidic or is adjusted to an acidic value, such as a pH of from 4 to 6, more preferably from 4 to 5, even more preferably of about 4.8.

Additionally or alternatively, the second composition includes a thickening agent to improve the ease of application to the teeth, such that even and effective coverage is more readily achieved. Suitable thickening agents include silicon dioxides, such as silica powder at a concentration preferably ranging from 3% to 10%, more preferably from 4% to 8%.

In some embodiments, the teeth-whitening kit of the invention comprises, in addition to the first and second compositions, a light source, such as a lamp, LED or laser.

In some embodiments, the teeth-whitening kit of the invention additionally comprises containers adapted to receive the first and second compositions. In certain embodiments, the kit includes a first container comprising the first composition that includes the oxidizing agent, and a second container comprising the second composition that includes the dyes, the catalyst and optionally the hydrophilic gelling agent, the stabilizer and the thickening agent. The containers may be light impermeable, air-tight and/or leak resistant. Exemplary containers include, but are not limited to, syringes, vials, or pouch. When a first composition and a second composition are included in the kit, it may be advantageous to provide the compositions in double-barrel syringes, including a mixer tip. The two compositions can then be dispensed and instantaneously mixed as the compositions exit the syringe.

A kit of the invention may also include directions for application. Additionally or alternatively, the kit can include apparatus for application (e.g., brushes or trays or both). The kit can also include charts or other information helpful in assessing the whitening effect desired and/or achieved by the methods and compositions of the disclosure.

Another aspect of the invention is a method for tooth whitening. The method comprises mixing the first and the second compositions as described above to obtain a teeth-whitening composition, then applying the teeth-whitening composition to at least one tooth and exposing the teeth-whitening composition on the at least one tooth to actinic light.

Light of different wavelengths, preferably between 430- 750 nm, can be used to increase the energy of the system and the speed of the reaction between the oxidizing agent the dyes and the catalyst, thereby accelerating the production of oxygen atoms and active species. Active oxygen species percolate within gaps in the enamel structure leading to changes in the optical disposition of the discoloration complexes, appearing as a whiter color.

The term "actinic light" is intended to mean light energy emitted from a specific light source (e.g. lamp, LED, or laser) and capable of being absorbed by matter (e.g. the dyes defined above). In a preferred embodiment, the actinic light is visible light.

In some embodiments, each tooth is exposed to actinic light for about 1-60 seconds. In other embodiments, each tooth is exposed to actinic light, for about 1-30 seconds. In other embodiments, each tooth is exposed to the actinic light for about 3-10 seconds, or for about 5- 10 seconds.

The following examples are provided by way of illustration only and are not intended to limit the scope of the invention as defined by the appended claims.

### EXAMPLE 1

### 1.1 Gel components:

| | |
|---|---|
| tot. gel | 100 g |
| carbopol | 1.81 g |
| EDTA | 0.67 g |
| Distilled water | 97.52 ml |

### Preparation of the gel:

EDTA was dissolved in 15 ml of distilled water at 50°C, the carbopol powder was poured in the remaining quantity of water and then the EDTA solution was added, mixing for 2 hours.

### 1.2 Cosmetic gel (2nd composition)

The gel prepared in 1.1 was then mixed with the dyes, the catalyst and the thickening agent to provide the "cosmetic gel".

### Composition of the cosmetic gel:

| | |
|---|---|
| tot. cosmetic gel | 100 g |
| gel of 1.1 | 90.7 g |
| E 102 | 2 g |
| E 122 | 2 g |
| KI | 4 ml |
| silica | 1 g |
| xanthan gum | 0.3 g |

### Preparation of the cosmetic gel:

The dyes (E 102 and E 122) were mixed in the gel prepared as disclosed in 1.1, then KI was added to the gel and mixed. Subsequently, the silica and the xanthan gum were added and mixed. The pH of the cosmetic gel was adjusted to about 5.5.

### 1.3 Oxidizing composition (1st composition)

| | |
|---|---|
| tot. gel | 100 g |
| urea | 29 g |
| carbopol | 3.6 g |
| EDTA | 0.6 g |
| Distilled water | 66.8 ml |

### Preparation of the oxidizing composition:

The EDTA was dissolved in 15 ml of distilled water at 50°C. the urea was dissolved in 30 ml of distilled water. The dissolved urea and the dissolved EDTA were mixed and then carbopol was added in the remaining quantity of water. The whole was mixed for about 2 hours, to provide a urea carbamide 29% gel. The he pH value was adjusted to about 5.

### EXAMPLE 2

### 2.1 Gel components:

| | |
|---|---|
| tot. gel | 100 g |
| carbopol | 1.81 g |
| EDTA | 0.67 g |
| Distilled water | 97.52 ml |

### Preparation of the gel:

EDTA was dissolved in 15 ml of distilled water at 50°C, the carbopol powder was poured in the remaining quantity of water and then the EDTA solution was added, mixing for 2 hours.

### 2.2 Medical device gel (2nd composition):

| | |
|---|---|
| tot. medical device gel | 100 g |
| gel of 2.1 | 90.4 g |
| E 102 | 2 g |
| E 122 | 2 g |
| KI | 2 ml |
| silica | 3 g |
| xanthan gum | 0.6 g |
| pH 5.5 | |

### 2.3 Oxidizing composition (1st composition)

Hydrogen peroxide 50%

### EXPERIEMNTAL TESTS

The inventors carried out an experimental test to compare different compositions: colours and peroxide; colours, peroxide and catalyst; colours, peroxide, catalyst and light; and peroxide, catalyst and light (wherein colors = E 102 and E 122 dyes).

The samples were prepared as follows:
1. Oxidizing agent gel + dyes
2. Oxidizing agent gel + dyes + light activation
3. Oxidizing agent gel + colours + catalyst + light activation
4. Oxidizing agent gel + catalyst + light activation.

When required, each sample was thoroughly mixed before activating with the light source. The light source used in the experiment was the Blue Fase light curing LED, by Ivoclar Vivadent (1200 mW/cm²).

Light activation follows for 60 seconds per sample.

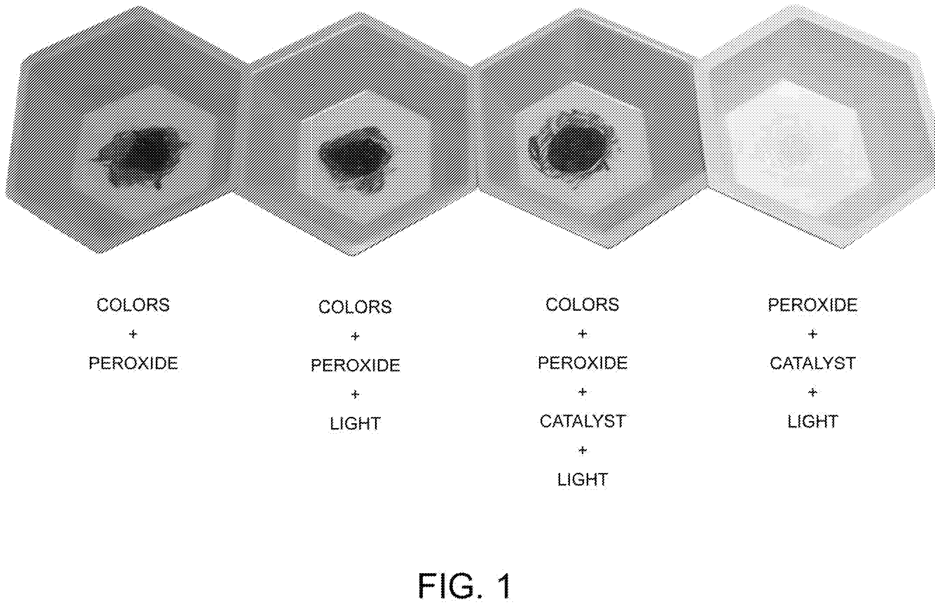

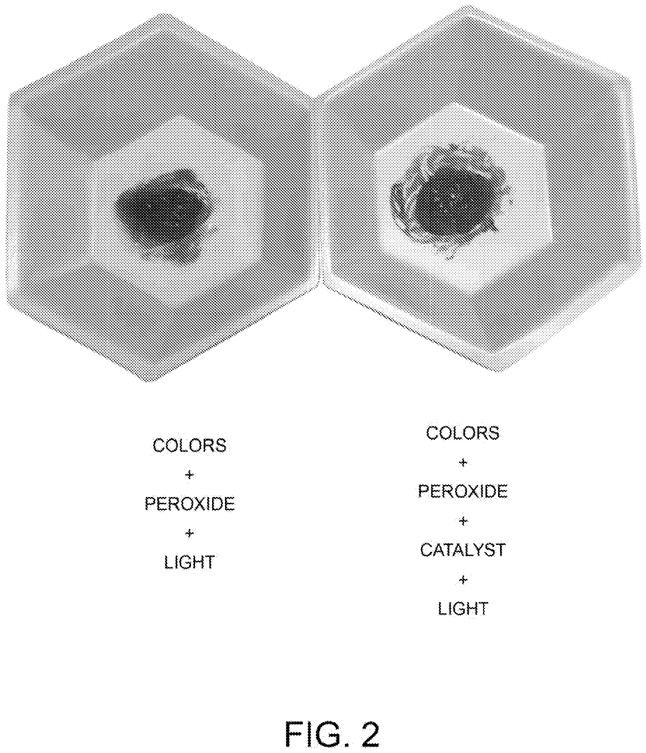

The inventors observed by the naked eye the generation of oxygen (bubbles) from each sample. Figure 1 is a picture showing all the samples for comparison after illumination with the LED (Blue Phase, Ivoclar-Vivadent) for 60 seconds. The first sample was not subjected to light activation. Figure 2 is a detail of Figure 1, where only samples 2 and 3 are shown.

Figures 1 and 2 show that the combination colors-peroxide-catalyst-light (sample 3), provides a significant amplification of oxygen production into the gel (bubbles). The synergy between the dyes complex, the catalyst and the light activation is qualitatively much higher than the other combinations used in the experimental test for comparison.

In conclusion, it was surprisingly found that the dyes-peroxide-catalyst + light activated gel (sample 3) generates massive quantities of O₂ in comparison to all the others. It is believed that the specific dye-catalyst complex formed upon mixing the components of the gel with light activation creates an extreme boosting of the peroxide decomposition and the oxygen radicals generation that leads to the increased bleaching effect on the tooth enamel.

## Claims

1. A kit for whitening teeth comprising a first composition and a second composition, the first composition comprising an oxidizing agent and the second composition comprising the dyes Azorubine and Tartrazine, and an oxidation catalyst.

2. The kit for whitening teeth according to claim 1, wherein the second composition comprises potassium iodide (KI) as the oxidation catalyst.

3. The kit for whitening teeth according to claim 2, wherein the second composition comprises about 90 to 180 ppm of potassium iodide (KI).

4. The kit for whitening teeth according to any of claims 1 to 3, wherein the first composition comprises hydrogen peroxide, carbamide peroxide or a combination thereof as the oxidizing agent.

5. The kit for whitening teeth according to claim 4, wherein the oxidizing agent is a 50% hydrogen peroxide aqueous solution.

6. The kit for whitening teeth according to claim 4, wherein the oxidizing agent is 29% carbamide peroxide gel.

7. The kit for whitening teeth according to claim 4, wherein the total content of the oxidizing agent is equivalent to a hydrogen peroxide content of about 6% by weight of the second composition.

8. The kit for whitening teeth according to any of claim 1 to 7, wherein the second composition comprises about 0.04 to 0.08 % by weight of Azorubine and about 0.04 to 0.08% by weight of Tartrazine.

9. The kit for whitening teeth according to claim 7, wherein the second composition comprises about 0.05 to 0.06 % by weight of Azorubine and about 0.05 to 0.06 % by weight of Tartrazine.

10. The kit for whitening teeth according to any of claims 1 to 9, wherein the second composition further comprises a stabilizing agent, a thickening agent, a hydrophilic gelling agent, or any combination thereof.

11. The kit for whitening teeth according to claim 10, wherein the thickening agent is silicon dioxide and/or fumed silica having a particle size less than one micron.

12. The kit for whitening teeth according to claim 10 or 11, wherein the hydrophilic gelling agent comprises polypropylene glycol, polyethylene glycol, propylene glycol, glycerol, or a vinyl polymer with active carboxyl groups such as carboxypolymethylene, or any combination thereof.

13. The kit for whitening teeth according to any of claims 1 to 12, wherein the pH of the second composition is from 4 to 6, more preferably from 4 to 5, even more preferably of about 4.8.

14. The kit for whitening teeth according to any of claims 1 to 13, further comprising a light source, preferably a lamp, an LED or a laser.

15. A method for whitening teeth comprising:
a) providing a kit for whitening teeth according to any of claims 1 to 13;
b) mixing the first and the second compositions of the kit thereby obtaining a teeth-whitening composition;
c) applying the teeth-whitening composition to at least one tooth; and
d) exposing the teeth-whitening composition on the at least one tooth to actinic light, preferably for about 1 to 60 seconds, more preferably for about 1 to 30 seconds.

## Patentansprüche

1. Kit zur Zahnaufhellung, umfassend eine erste Zusammensetzung und eine zweite Zusammensetzung, wobei die erste Zusammensetzung ein Oxidationsmittel umfasst und die zweite Zusammensetzung die Farbstoffe Azorubin und Tartrazin und einen Oxidationskatalysator umfasst.

2. Kit zur Zahnaufhellung nach Anspruch 1, wobei die zweite Zusammensetzung Kaliumiodid (KI) als Oxidationskatalysator umfasst.

3. Kit zur Zahnaufhellung nach Anspruch 2, wobei die zweite Zusammensetzung ungefähr 90 bis 180 ppm Kaliumiodid (KI) umfasst.

4. Kit zur Zahnaufhellung nach einem der Ansprüche 1 bis 3, wobei die erste Zusammensetzung Wasserstoffperoxid, Karbamidperoxid oder eine Kombination davon als Oxidationsmittel umfasst.

5. Kit zur Zahnaufhellung nach Anspruch 4, wobei das Oxidationsmittel eine wässrige Lösung mit 50 % Wasserstoffperoxid ist.

6. Kit zur Zahnaufhellung nach Anspruch 4, wobei das Oxidationsmittel ein Gel mit 29 % Karbamidperoxid ist.

7. Kit zur Zahnaufhellung nach Anspruch 4, wobei der Gesamtgehalt an Oxidationsmittel gleich einem Gehalt an Wasserstoffperoxid von ungefähr 6 Gew.-% der zweiten Zusammensetzung ist.

8. Kit zur Zahnaufhellung nach einem der Ansprüche 1 bis 7, wobei die zweite Zusammensetzung ungefähr 0,04 bis 0,08 Gew.-% Azorubin und ungefähr 0,04 bis 0,08 Gew.-% Tartrazin umfasst.

9. Kit zur Zahnaufhellung nach Anspruch 7, wobei die zweite Zusammensetzung ungefähr 0,05 bis 0,06 Gew.-% Azorubin und ungefähr 0,05 bis 0,06 Gew.-% Tartrazin umfasst.

10. Kit zur Zahnaufhellung nach einem der Ansprüche 1 bis 9, wobei die zweite Zusammensetzung ferner einen Stabilisator, ein Verdickungsmittel, einen hydrophilen Gelbildner oder eine Kombination davon umfasst.

11. Kit zur Zahnaufhellung nach Anspruch 10, wobei das Verdickungsmittel Siliciumdioxid und/oder pyrogene Kieselsäure mit einer Partikelgröße von weniger als ein Mikrometer ist.

12. Kit zur Zahnaufhellung nach Anspruch 10 oder 11, wobei der hydrophile Gelbildner Polypropylenglycol, Polyethylenglycol, Propylenglycol, Glycerin oder ein Vinylpolymer mit aktiven Carboxylgruppen wie Carboxypolymethylen oder eine Kombination davon umfasst.

13. Kit zur Zahnaufhellung nach Anspruch 1 bis 12, wobei der pH-Wert der zweiten Zusammensetzung 4 bis 6, mehr bevorzugt 4 bis 5, noch mehr bevorzugt ungefähr 4,8 beträgt.

14. Kit zur Zahnaufhellung nach einem der Ansprüche 1 bis 13, umfassend ferner eine Lichtquelle, vorzugsweise eine Lampe, eine LED oder einen Laser.

15. Verfahren zur Zahnaufhellung, umfassend:
a) Bereitstellen eines Kits zur Zahnaufhellung nach einem der Ansprüche 1 bis 13,
b) Mischen der ersten und der zweiten Zusammensetzung des Kits, wodurch eine Zusammensetzung zur Zahnaufhellung erhalten wird;
c) Aufbringen der Zusammensetzung zur Zahnaufhellung auf mindestens einen Zahn; und
d) aktinischem Licht Aussetzen der Zusammensetzung zur Zahnaufhellung auf mindestens einem Zahn, vorzugsweise für ungefähr 1 bis 60 Sekunden, mehr bevorzugt für ungefähr 1 bis 30 Sekunden.

## Revendications

1. Kit pour le blanchiment des dents comprenant une première composition et une seconde composition, la première composition comprenant un agent oxydant et la seconde composition comprenant les colorants Azorubine et Tartrazine, ainsi qu'un catalyseur d'oxydation.

2. Kit pour le blanchiment des dents selon la revendication 1, où la seconde composition comprend de l'iodure de potassium (KI) comme catalyseur d'oxydation.

3. Kit pour le blanchiment des dents selon la revendication 2, où la seconde composition comprend de 90 à 180 ppm environ d'iodure de potassium (KI).

4. Kit pour le blanchiment des dents selon l'une quelconque des revendications de 1 à 3, où la première composition comprend comme agent oxydant du peroxyde d'hydrogène, du peroxyde de carbamide ou une leur combinaison.

5. Kit pour le blanchiment des dents selon la revendication 4, où l'agent oxydant est une solution aqueuse à 50% de peroxyde d'hydrogène.

6. Kit pour le blanchiment des dents selon la revendication 4, où l'agent oxydant est un gel de peroxyde de carbamide à 29%.

7. Kit pour le blanchiment des dents selon la revendication 4, où le contenu total de l'agent oxydant est équivalent à un contenu de peroxyde d'hydrogène d'environ le 6% en poids de la seconde composition.

8. Kit pour le blanchiment des dents selon l'une quelconque des revendications de I à 7, où la seconde composition comprend de 0,04 à 0,08% environ en poids d'Azorubine et de 0,04 à 0,08% environ en poids de Tartrazine.

9. Kit pour le blanchiment des dents selon la revendication 7, où la seconde composition comprend de 0,05 à 0,06% environ en poids d'Azorubine et de 0,05 à 0,06% environ en poids de Tartrazine.

10. Kit pour le blanchiment des dents selon l'une quelconque des revendications de I à 9, où la seconde composition comprend également un agent stabilisateur, un agent épaississant, un agent gélifiant hydrophile ou une leur quelconque combinaison.

11. Kit pour le blanchiment des dents selon la revendication 10, où l'agent épaississant est du dioxyde de silicium et/ou de l'acide silicique pyrogène formé de particules dont la taille est inférieure à un micron.

12. Kit pour le blanchiment des dents selon la revendication 10 ou 11, où l'agent gélifiant hydrophile comprend du polypropylène glycol, du polyéthylène glycol, du propylène glycol, du glycérol ou un polymère vinylique avec des groupes carboxyles actifs tels que le carboxypolyméthylène ou une leur quelconque combinaison.

13. Kit pour le blanchiment des dents selon l'une quelconque des revendications de I à 12, où le pH de la seconde composition est de 4 à 6, plus préférablement de 4 à 5, encore plus préférablement d'environ 4,8.

14. Kit pour le blanchiment des dents selon l'une quelconque des revendications de I à 13, comprenant également une source lumineuse, préférablement une lampe, une DEL ou un laser.

15. Méthode de blanchiment des dents comprenant :
a) fournir un kit pour le blanchiment des dents selon l'une quelconque des revendications de 1 à 13 ;
b) mélanger la première et la seconde compositions du kit pour obtenir ainsi une composition de blanchiment des dents ;
c) appliquer la composition de blanchiment des dents à au moins une dent ; et
d) exposer la composition de blanchiment des dents sur ladite au moins une dent à la lumière actinique, préférablement pendant environ de 1 à 60 secondes, plus préférablement pendant environ de 1 à 30 secondes.
